# EUROPEAN PATENT APPLICATION

(11) **EP 2 842 594 A1**
(43) Date of publication of application: **04.03.2015**
(21) Application number: 13290209.9
(22) Date of filing: 03.09.2013
(51) Int. Cl.: A61M 25/06

(54) **Access sheath**

(71) Applicant: Coloplast A/S, 3050 Humlebaek (DK)
(72) Inventor: Callede, David, 24200 Sarlat la Caneda (FR); Pascal, Laurent, 24200 Sarlat La Caneda (FR)

(57) **Abstract**

The present invention concerns an access sheath (10) intended for positioning a tool in a working position during an intervention on a patient. Said access sheath comprises a hollow guide tube (11) comprising a longitudinal hole (12). The access sheath is characterized in that said hollow guide tube comprises at least an opening (14) positioned on the periphery of the guide tube and connecting the outside of the access sheath with said longitudinal hole.

## Description

### TECHNICAL FIELD

The present invention concerns an access sheath intended for positioning a tool in a working position during an intervention on a patient, said access sheath comprising a hollow guide tube comprising a longitudinal hole.

This kind of access sheath is used together with a tool by surgeons to enable them to make an intervention in areas of the human body which can be difficult to access. These access sheath/tool assemblies are used, among others, in urology. More particularly, the access sheath of the present invention is used for urological interventions in particular in the kidney.

Such an access sheath can be used on its own, in particular for inserting tools into an area of the body where an intervention has to take place. The access sheath of the invention can also be part of an assembly further comprising a dilator that may be inserted into the access sheath and a guide for guiding the access sheath during its insertion into the patient's body.

### BACKGROUND ART

When a surgeon wants to gain access to a kidney, if surgical intervention is not an option, he has to introduce an access sheath inside the urethra starting from a natural route of entry, pass the bladder and then go up inside the ureter to reach the kidney.

The positioning of such an access sheath can be performed by several methods. According to a first method, a first radio-opaque guide is driven up to the bladder by the aid of a cystoscope previously introduced inside the bladder. Then, with the aid of the cystoscope, the urethral meatus is targeted in order to insert the guide into the urethra. The guide is a generally sheathed nickel, titanium and/or stainless steel alloy lead.

After the setting up of this first guide, a radio-opaque double channel urethral probe or dilator is engaged by one of its two channels onto the guide and is driven up to the urethra. Through the other channel of the urethral probe, a second guide is inserted until it reaches the urethra. Then the urethral probe is removed, leaving behind only the two guides : a working guide and a security guide, this latter being fixed to the patient.

During these first steps, the radio-opaque components have been visualized to check their positions.

The access sheath being threaded onto a dilator projecting forwards outside the sheath, the working guide is engaged into the access sheath through the dilator channel. The access sheath is driven up to a position between the bladder and the kidney, but nearer the bladder. The dilator and the working guide are removed to leave behind in place only the access sheath, and near it, the security guide that can be used in case of difficulty.

According to a second, more efficient method, the positioning of the access sheath requires the use of only one guide and a dilator comprising at least a hole at its distal end, i.e. the end that is in the area of the intervention when the access sheath is in use. The dilator further comprises an opening connected to the hole by a channel comprising a slit on its periphery. Said opening is positioned outside of the access sheath when the access sheath and the dilator are assembled. Such an access sheath/dilator assembly is described in the patent application WO 2009/127216.

As in the previous embodiment, a guide is driven up to the bladder of the patient. The access sheath/dilator assembly is engaged with the guide, this guide entering the hole at the distal end of the dilator, following the channel and exiting the dilator through the opening.

The access sheath/dilator assembly is positioned by following the guide until it reaches the final position. The dilator is then pulled out of the access sheath. This has the effect of applying strength on the guide which in turn opens the slit of the dilator and releases the guide from the dilator. Thus, the access sheath is ready for use, with the guide following the access sheath on the external side.

In order to enable the surgeon to work efficiently, the inner diameter of the access sheath should be as large as possible. However, as the urethra has a restricted cross section, in order to avoid injuries and pain to the patient, the inner diameter of the access sheath should not be too large. In any case, the access sheath usually fills the urethra. This causes a problem as the urine contained in the bladder cannot easily be evacuated.

In order to solve this problem, the external diameter of the access sheath may be reduced. This implies that smaller tools must be used. This can pose a problem for different reasons. For example, if the operation consists in removing stones from the kidney, these stones are fractured into pieces that are smaller than the inner diameter of the access sheath. The smaller the inner diameter of the access sheath is, the higher is the number of stone fragments to be removed. This lengthens the duration of the operation, which is not optimal.

The miniaturization of the tools can also lead to poorer quality tools, for example if these tools comprise laser beams devices or cameras. For an equivalent quality, miniaturized tools usually imply higher costs. A reduction of the inner diameter of the access sheath also complicates the work of the surgeon.

For these reasons, the miniaturization is not an interesting option in most of the cases and it is often desirable to work with an access sheath that is as large as possible without inducing pain or injuries to the patient.

The object of this invention is to provide an access sheath having a diameter sufficiently large to enable a surgeon to work with efficiency while enabling the evacuation of the urine contained in the bladder.

According to this invention, the working conditions for the surgeon are optimal and the comfort for the patient is as good as possible.

### DISCLOSURE OF INVENTION

The object of the invention is achieved by an access sheath as described in the preamble and **characterized in that** said hollow guide tube comprises at least an opening positioned on the periphery of the guide tube and connecting the outside of the access sheath with said longitudinal hole.

According to the present invention, the access sheath can be used in cooperation with different kinds of tools such as for example an endoscope, tools for grasping objects, tools for cutting tissues, tools with laser beams... These tools are usually introduced in the access sheath once this access sheath is in place within the body of the patient, an end of said access sheath being placed in the place where the intervention takes place.

The access sheath comprises means for enabling the evacuation of the urine during its use. According to this means, the surgeon can regularly or continuously empty the bladder. This is particularly useful during long interventions during which the bladder can be filled, causing unpleasant feeling to the patient.

### BRIEF DESCRIPTION OF DRAWINGS

The present invention and its advantages will be better understood with reference to the enclosed drawing and to the detailed description of specific embodiments, in which :
- Fig. 1 is a schematic view a first embodiment of an access sheath according to the present invention;
- Fig. 2 is a view similar to Fig. 1, of a second embodiment of the invention;
- Fig. 3 illustrates a third embodiment of the access sheath of the invention;
- Fig. 4 is a partial cross section view of another detail of the access sheath of the invention; and
- Fig. 5 is a cross section view of a detail of the access sheath of the invention.

### BEST MODES FOR CARRYING OUT THE INVENTION

With reference to the figures, the access sheath 10 of the present invention is intended for receiving a tool (not represented) that will be used in an area of a body which is difficult to access. This tool can be an endoscope or an other device for taking pictures of the inner part of the patient's body, tools for grasping objects, tools for cutting patient's tissues, tools for delivering drugs, liquids or other products or tools comprising laser beams for example. The access sheath 10 comprises a hollow guide tube 11 comprising a longitudinal hole 12 for receiving and guiding the tool. The access sheath further comprises a bucket 13 having a general shape of a funnel with a hole coinciding with the hole 12 of the guide tube 11.

The access sheath of the invention further comprises openings 14 in the periphery of the guide tube and connecting the outside of the access sheath with the inner longitudinal hole 12.

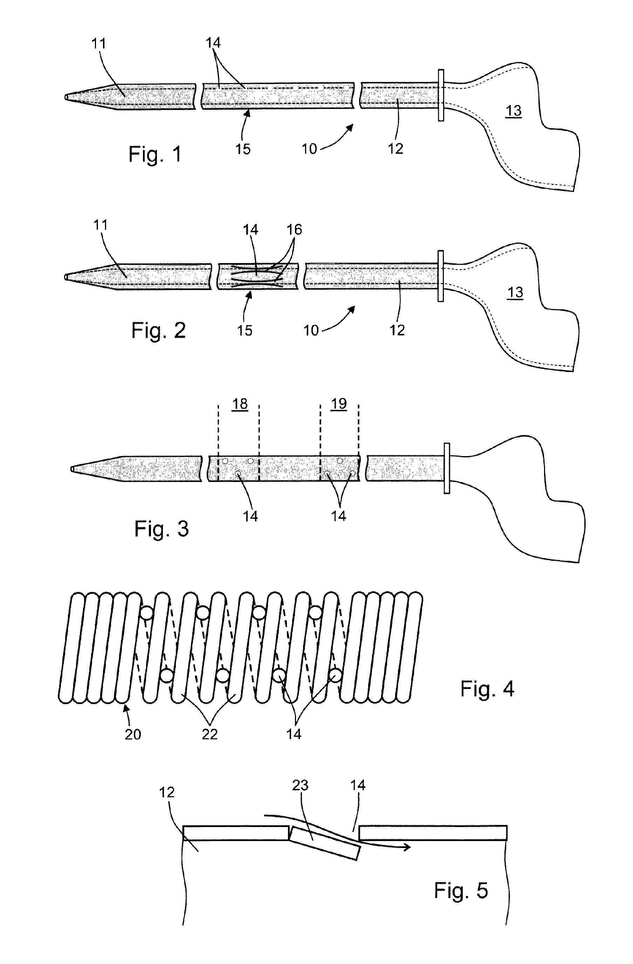

According to the embodiment illustrated by Fig. 1, the openings 14 are formed of holes disposed in a limited area 15 of the guide tube 11. This area 15 is intended to be inside the bladder when the access sheath is used on a patient and placed in a working position.

In this embodiment, liquid can flow from the outside of the access sheath to the inside. This is particularly useful for emptying the bladder of urine. This urine can flow through the openings 14, be collected in the longitudinal hole 12 of the access sheath 10 and be collected through the bucket 13.

As the openings 14 are formed in an area of the guide tube 11 which is in the bladder while the access sheath is in a working position, only the liquid contained in the bladder is removed.

In the embodiment of Fig. 2, the openings 14 are formed of slits 16. According to this embodiment, at least a part of the material between two slits 16 is elastic. A pressure applied on material disposed between two slits if deformed in a way to open the slits and to enable liquid to flow from the outside of the access sheath to the inside of the longitudinal hole 12.

In this embodiment, it is possible to configure the slits 16 in such a way that, in the absence of pressure, the openings are closed. When pressure is applied on the slits, for example due to the presence of liquid on the external periphery of the access sheath 10, the slits are deformed to open the opening 14 and to enable the liquid to flow through these openings to the longitudinal hole. It is also possible to provide slits 16 that open only when pressure is applied from the outside of the access sheath towards the inside. If pressure is applied from inside, the openings 14 remain closed. This can be achieved by a specific shape of the slits, by using a specific material or a prestress for example.

In the embodiments of Fig. 1 and 2, the openings 14 are located in one area 15 which is usually in the bladder when the access sheath is in a working position ready for use. In the embodiment of Fig. 3, the access sheath contains two areas 18, 19 of openings 14. This embodiment can be interesting, in particular as it enables using a same type of access sheath with patients of different sizes. It further enables working at different locations within a patient's body while having at least one area 18, 19 containing openings 14 positioned in the bladder of the patient.

Fig. 4 represents a detail of the access sheath 10 according to the present invention. As it is well known, an access sheath may comprise a metallic coil 20 coated with a synthetic material. This ensures the flexibility as well as the rigidity of the access sheath. Hence, an access sheath should be rigid in a longitudinal direction and flexible in a direction which is perpendicular to that longitudinal direction. This feature is well achieved by a helical coil or helical spring.

In the embodiment of Fig. 4, some turns 22 of the helical coil are spread out so that the openings 14 fall between two turns of the coil. The separation of the turns could be done for example by winding a metallic wire around a chuck, this chuck comprising pins at the places where the openings must be present.

It should be noted that the helical coil 20 could be made of a synthetic material instead of metal. It should further be noted that the helical coil could also be replaced by a series of rings that are parallel to each other. In this case, the space between two adjacent rings could be greater in the area where openings are made than in other areas without openings. It is also possible to realize an access sheath without a "core" such as a helical spring or rings.

Fig. 5 is a cross section view of a part of the access sheath, said part containing an opening 14. According to the embodiment illustrated, the opening cooperates with a cap 23 that is articulated on one side of this opening. The articulation is placed at the inner side of the access sheath. Thus, if pressure is applied from the outside of the access sheath 10, the cap 23 opens and liquid can flow towards the inside of this access sheath, in the longitudinal hole 12. On the contrary, if pressure is applied towards the outside of the access sheath, the caps 23 tend to close the openings 14 and liquid cannot flow from the inside to the outside.

This embodiment is useful for example for dispensing a liquid at a given time, to the patient by using the longitudinal hole 12 of the access sheath and in particular the distal end of this access sheath and for emptying the bladder during another phase of the intervention.

The present invention simplifies the work of the surgeon and enhances the comfort of the patient as it provides an easy way of emptying the patient's bladder during an intervention.

## Claims

1. Access sheath intended for positioning a tool in a working position during an intervention on a patient, said access sheath (10) comprising a hollow guide tube (11) comprising a longitudinal hole (12), **characterized in that** said hollow guide tube (11) comprises at least an opening (14) positioned on the periphery of the guide tube and connecting the outside of the access sheath with said longitudinal hole (12).

2. Access sheath according to claim 1, **characterized in that** said at least one opening (14) is positioned on the guide tube (11) in a way to be in a bladder of said patient when the access sheath (10) is in position for use.

3. Access sheath according to claim 1, **characterized in that** said guide tube (11) comprises a plurality of openings (14) positioned in a limited area (15, 18, 19) of the guide tube (11).

4. Access sheath according to claim 1, **characterized in that** said guide tube (11) comprises a plurality of openings (14) positioned in at least two distinct limited areas (18, 19) of the guide tube (11).

5. Access sheath according to claim 1, **characterized in that** the openings (14) are holes.

6. Access sheath according to claim 1, **characterized in that** the openings (14) comprise slits (16).

7. Access sheath according to claim 4, **characterized in that** the openings (14) cooperate with caps (23).

8. Access sheath according to claim 7, **characterized in that** a cap (23) comprises an articulation for opening the opening (14) when a pressure is applied from the outside of the access sheath (10) towards the longitudinal hole (12) and for closing said opening (14) when a pressure is applied from the inside of the guide tube (11) towards the outside.

9. Access sheath according to claim 6, **characterized in that** material between at least two slits (16) is elastic.

10. Access sheath according to claim 9, **characterized in that** the elastic material is configured for opening the opening (14) when a pressure is applied from the outside of the access sheath (10) towards the longitudinal hole (12) and for closing said openings (14) when a pressure is applied from the inside of the guide tube (11) towards the outside.

11. Access sheath according to claim 1, **characterized in that** it comprises an helical coil (20) coated by a polymer material.

12. Access sheath according to claim 11, **characterized in that** at least some of the turns (22) of the helical coil (20) are separated and **in that** the openings (14) are positioned in the separation between the turns (22) of the metallic coil (20).
